# EUROPEAN PATENT APPLICATION

(11) **EP 1 574 848 A1**
(43) Date of publication of application: **14.09.2005**
(21) Application number: 03782815.9
(22) Date of filing: 19.12.2003
(51) Int. Cl.: G01N 27/327, G01N 27/416

(54) **BIOSENSOR**

(30) Priority: 20.12.2002 JP 2002370331
(71) Applicant: MATSUSHITA ELECTRIC INDUSTRIAL CO., LTD., Osaka 571-8501 (JP)
(72) Inventor: NAKAMINAMI, Takahiro, Toyonaka-shi, Osaka 560-0033 (JP); IKEDA, Shin, Katano-shi, Osaka 576-0022 (JP); YOSHIOKA, Toshihiko, Hirakata-shi, Osaka 573-0035 (JP)
(74) Representative: Gassner, Wolfgang
(86) International application number: PCT/JP2003/016303
(87) International publication number: WO 2004/057321

(57) **Abstract**

The present invention relates to a biosensor which comprises an electrode system including at least one pair of electrodes, at least one insulating base plate for supporting the electrode system, a first reaction layer provided at least on a working electrode of the electrode system, including an organic compound having a functional group capable of bonding or being adsorbed to an electrode and a hydrophobic hydrocarbon group, a second reaction layer provided on the first reaction layer, including an amphiphilic lipid capable of bonding or being adsorbed to a hydrophobic portion of the first reaction layer, and a reagent system carried in a two-component membrane composed of the first and second reaction layers, including at least membrane-binding type pyrroquinoline quinone-dependent glucose dehydrogenase and an electron mediator.

## Description

### TECHNICAL FIELD

The present invention relates to a biosensor for measuring a substrate (a substance to be measured) contained in a sample solution. More particularly, the present invention relates to a biosensor for measuring the concentration of glucose contained in a sample solution.

### BACKGROUND ART

Measurement error in measured values (e.g., a substrate concentration, etc.) obtained by a biosensor is caused by the influence of substances other than a substance to be measured (a substrate) contained in a sample solution.

For example, when a current detection type electrochemical sensor is used to measure the glucose concentration of a blood sample, oxidizable chemical substances, such as ascorbic acid (vitamin C), uric acid, acetaminophen and the like, which are contained in blood, are electrochemically oxidized to generate a current at an electrode (working electrode) of the sensor. This current is superposed on a current caused by glucose, so that a positive error occurs in the measured value of glucose concentration.

The blood concentrations of these compounds vary from individual to individual, and even in the same individual, vary from day to day. Therefore, it is difficult to predict and correct measurement errors which will occur.

Chemical substances, which generate such errors, are called oxidizable interfering compounds (abbreviated as OIC). Various techniques have been tried to remove the influences of these chemical substances in the art.

One of such techniques is disclosed in US Patent No. 6,340,428. In this technique, a third electrode for measuring OIC is provided on a base plate of a biosensor in addition to a working electrode and a counter electrode thereof so as to correct the influence of OIC.

As a method for removing the influence of OIC, a method and biosensor have been developed, in which a film for blocking OIC from diffusing to a working electrode is provided on the working electrode, thereby suppressing a current caused by OIC. As an example of such a biosensor, a biosensor using poly(o-phenylenediamine) film is disclosed in Wang, J. et al., "Enhanced selectivity and sensitivity of first-generation enzyme electrodes based on the coupling of rhodinized carbon paste transducers and permselective poly(o-phenylenediamine) coatings", Electroanalysis, Vol. 8, 1996, pp. 1127-1130.

Blood cells and peptides, such as proteins and the like, which are contained in blood, are easily adsorbed onto an electrode surface. Therefore, current interference is also caused by adsorption of these peptides onto the electrode surface. When these adsorptive interfering compounds (abbreviated as AIC) are adsorbed onto an electrode (working electrode) of a glucose sensor, the effective electrode area is decreased. As a result, a current based on a redox reaction, in which glucose is involved, is reduced, resulting in a negative error in measurement. The degree of a reduction in the current varies depending on the degree of adsorption of AIC onto the electrode surface. The adsorption degree varies depending on the AIC concentration of a sample solution. Therefore, it is difficult to predict the degree of current reduction and correct an error which will occur.

Various techniques have been tried to remove the influence of AIC. For example, a method is disclosed, in which a filter paper for separating blood cells is provided on an electrode system of a biosensor, thereby removing blood cells or the like physically and efficiently (see, for example, US Patent No. 6,033,866).

Alternatively, adsorption of AIC, such as blood cells, proteins and the like, is suppressed by applying a hydrophilic polymer, such as carboxymethylcellulose or the like, onto a surface of an electrode system of a biosensor (see, for example, Japanese Laid-Open Patent Publication No. 3-202764).

### DISCLOSURE OF THE INVENTION

In the above-described conventional biosensors, however, when a fluid containing OIC and AIC is used as a sample solution, the suppression of oxidation of OIC at the working electrode and the suppression of adsorption of AIC onto the electrode (working electrode) surface are not necessarily completely achieved. Therefore, measurement errors still occur in measurement of substrate concentration, so that the substrate concentration of a sample solution is estimated to be lower or higher than the actual substrate concentration in the sample solution. Alternatively, correction of a current caused by oxidation of OIC and filtration of AIC lead to more complex sensor structure.

The present invention is provided to solve the above-described problems in the conventional art. An object of the present invention is to provide a simple structure biosensor, which is capable of measuring a substrate in a sample solution quickly and with high precision while removing measurement errors of the biosensor.

In order to solve the above-described problems, the present invention provides a biosensor for measuring a substrate concentration of a sample solution with high accuracy and reproducibility. The biosensor comprises an electrode system including at least one pair of electrodes, at least one insulating base plate for supporting the electrode system, a first reaction layer provided at least on a working electrode of the electrode system, and a second reaction layer provided on the first reaction layer.

In the biosensor, the first reaction layer includes an organic compound having a functional group capable of bonding or being adsorbed to an electrode and a hydrophobic hydrocarbon group. The second reaction layer includes an amphiphilic lipid capable of bonding or being adsorbed to a hydrophobic portion of the first reaction layer.

The biosensor of the present invention further comprises a reagent system carried in a two-component membrane composed of the first and second reaction layers. The reagent system includes at least membrane-binding type pyrroquinoline quinone-dependent glucose dehydrogenase and an electron mediator.

In a preferable embodiment, the first reaction layer and the second reaction layer each form a monolayer.

In a preferable embodiment, the organic compound is a compound represented by the following general formula (1), (2) or (3) ;

HS-(CH₂)ₙ-X (1)

X- (CH₂)ₙ-S-S- (CH₂)ₙ-X (2)

S-(CH₂)ₙ-X (3)

where n is an integer of 1 to 20, and X is a methyl group, a benzyl group, an aminobenzyl group, a carboxybenzyl group or a phenyl group. More preferably, n is an integer of 5 to 15.

In a preferable embodiment, the amphiphilic lipid is L-α-phosphatidylcholine, β-oleoyl-γ-palmitoyl.

In a preferable embodiment, the electron mediator is 1-methoxy-5-methylphenazinium.

In a preferable embodiment, the working electrode contains gold, platinum or palladium.

In a preferable embodiment, a counter electrode of the electrode system contains none of gold, platinum and palladium.

In a preferable embodiment, the entirety of a surface of the working electrode of the electrode system and a portion of a surface of a counter electrode are covered with the two-component membrane. More preferably, the area of a portion of the counter electrode, which is not covered with the two-component membrane, is larger than the area of the working electrode.

In a preferable embodiment, only the surface of the working electrode of the electrode system is covered with the two-component membrane.

In a preferable embodiment, the pair of electrodes are supported on respective surfaces of two insulating base plates, the surfaces of the two insulating plates facing each other in a manner which forms a supply path for the sample solution therebetween.

In a preferable embodiment, the reagent system further includes a pH buffering agent.

According to another aspect, the present invention provides a method for fabricating a biosensor. The biosensor fabrication method of the present invention comprises the steps of forming an electrode system including at least one pair of electrodes on an insulating base plate, providing a first reaction layer at least on a working electrode of the electrode system, wherein a solution containing an organic compound having a functional group capable of bonding or being adsorbed to an electrode and a hydrophobic hydrocarbon group is made contact with the working electrode, and providing a second reaction layer on the first reaction layer, wherein a solution containing an amphiphilic lipid is made contact with the first reaction layer. At least one of the solution containing the organic compound and the solution containing the amphiphilic lipid contains an electron mediator. The solution containing the amphiphilic lipid further contains membrane-binding type pyrroquinoline quinone-dependent glucose dehydrogenase.

In a preferable embodiment of the biosensor fabrication method of the present invention, the first reaction layer and the second reaction layer each form a monolayer.

In a more preferable embodiment, the organic compound is a compound represented by the following general formula (1), (2) or (3);

HS-(CH₂)ₙ-X (1)

X-(CH₂)ₙ-S-S-(CH₂)ₙ-X (2)

S-(CH₂)ₙ-X (3)

where n is an integer of 1 to 20, and X is a methyl group, a benzyl group, an aminobenzyl group, a carboxybenzyl group or a phenyl group. More preferably, n is an integer of 5 to 15.

In a preferable embodiment of the biosensor fabrication method of the present invention, the amphiphilic lipid is L-α-phosphatidylcholine, β-oleoyl-γ-palmitoyl.

In a preferable embodiment of the biosensor fabrication method of the present invention, the electron mediator is 1-methoxy-5-methylphenazinium.

In a preferable embodiment of the biosensor fabrication method of the present invention, the working electrode contains gold, platinum or palladium.

In a preferable embodiment of the biosensor fabrication method of the present invention, a counter electrode of the electrode system contains none of gold, platinum and palladium.

In a preferable embodiment of the biosensor fabrication method of the present invention, the entirety of a surface of the working electrode of the electrode system and a portion of a surface of a counter electrode are covered with the first and second reaction layers. More preferably, the area of a portion of the counter electrode, which is not covered with the first and second reaction layers, is larger than the area of the working electrode.

In a preferable embodiment of the biosensor fabrication method of the present invention, only the surface of the working electrode of the electrode system is covered with the first and second reaction layers.

In a preferable embodiment of the biosensor fabrication method of the present invention, the pair of electrodes are supported on respective surfaces of two insulating base plates, the surfaces of the two insulating plates facing each other in a manner which forms a supply path for the sample solution therebetween.

In a preferable embodiment of the biosensor fabrication method of the present invention, at least one of the solution containing the organic compound and the solution containing the amphiphilic lipid further contains a pH buffering agent.

The present invention is based on the present inventors' finding that pyrroquinoline quinone-dependent glucose dehydrogenase (hereinafter abbreviated as PQQ-GDH) can be stably fixed on an electrode surface on a base plate by utilizing a two-component membrane composed of an organic compound film containing sulfur atoms, which are bound to the electrode surface, and an amphiphilic lipid film provided on the organic compound film.

As a means for effectively preventing an influence of OIC on a redox reaction occurring on the electrode surface and an influence of AIC adsorbed on the electrode surface, and selectively performing only a redox reaction between the electrode and glucose, the present invention employs the two-component membrane provided on the electrode and an enzyme binding or adsorbed to the two-component membrane, i.e., membrane-binding type PQQ-GDH. To the present inventors' knowledge, there had been no precedent before the present invention, in which such a two-component membrane and membrane-binding type PQQ-GDH are applied to a glucose sensor.

Thus, by using the biosensor electrode, in which PQQ-GDH is fixed in the two-component membrane formed on the electrode, and using an appropriate electron mediator in combination with PQQ-GDH in the two-component membrane, a systemwhich allows highly selective electrochemical oxidation of glucose (substrate) can be achieved.

In general, enzymes which catalyze a selective oxidation reaction of glucose are roughly divided into glucose oxidase (GOx) and glucose dehydrogenase (GDH) according to the reaction scheme. Further, it is known that GDH includes nicotinamide adenine dinucleotide (NAD)-dependent GDH (NAD-GDH) and PQQ-GDH.

Further, it is known that PQQ-GDH includes water-soluble type PQQ-GDH, which is conventionally known to be applicable to biosensors, and in addition, membrane-binding type PQQ-GDH (see, for example, Oubrie et al., The EMBO Journal, Vol. 18, No. 19, pp. 5187-5194, 1999; and Matsushita et al., Biochemistry, 1989, 28(15), 6276-80). Membrane-binding type PQQ-GDH refers to PQQ-GDH which has a hydrophobic domain on a surface of the protein molecule thereof, the hydrophobic domain binding via hydrophobic interaction to, for example, the hydrophobic portion of a lipid bilayer, such as cell membrane, with high af finity to achieve stable fixation to biological membrane in organisms. In contrast, there is no known membrane-binding type GOx and NAD-GDH, though only water-soluble ones are currently available.

Among these enzymes, i.e., GOx, NAD-GDH and PQQ-GDH, membrane-binding type PQQ-GDH is the most suitable for fixation to a two-component membrane including a sulfur-containing organic compound/amphiphilic lipid as major components in the present invention. This is because membrane-binding ability can cause an enzyme, which catalyzes a selective oxidation reaction of glucose, to stably bind or be adsorbed to the hydrophobic portion of the two-component membrane. Water-soluble enzyme molecures have substantially no hydrophobic domain on the surface thereof, and therefore, have difficulty in binding to the hydrophobic portion of the two-component membrane with high affinity.

The present invention provides a simple structure biosensor for a fluid containing OIC and AIC as a sample solution, capable of removing measurement errors caused by oxidation of OIC at the working electrode and measurement errors caused by adsorption of AIC on the electrode (working electrode) surface and measuring a substrate in the sample solution quickly and with high precision.

The biosensor of the present invention inhibits oxidation of OIC at the working electrode and adsorption of AIC on the electrode (or the working electrode) surface by means of the two-component membrane and achieves electron transport between an enzyme used and the working electrode. As a result, it is possible to remove conventionally problematic measurement errors in measuring substrate concentration. Moreover, it is noteworthy in the present invention, oxygen accepts no electron from PQQ-GDH. Therefore, the influence of dissolved oxygen on glucose oxidation can be advantageously avoided, which otherwise occurs when glucose oxidase is used as in conventional techniques.

### BRIEF DESCRIPTION OF THE DRAWINGS

FIG. 1 is an exploded perspective view of a biosensor according to one embodiment of the present invention, where a reagent system is omitted.
FIG. 2 is a longitudinal cross-sectional view of a biosensor according to one embodiment of the present invention.
FIG. 3 is a schematic diagram showing a portion of a production scheme of a biosensor according to one embodiment of the present invention and an outline of the principle of the present invention.
FIG. 4 is a diagram showing an exemplary electron mediator used in a biosensor of the present invention: (A) electron mediators (indicated by A) are bound to a polymer backbone; and (B) electron mediators (entirety) are linked to one another to form a polymer chain.
FIG. 5 is a diagram showing a Hct dependency of a current obtained in one embodiment of the present invention.
FIG. 6 is a diagram showing an ascorbic acid concentration dependency of a current obtained in one embodiment of the present invention.
FIG. 7 is an exploded perspective view of a biosensor according to one embodiment of the present invention, where a reagent system is omitted.

### BEST MODE FOR CARRYING OUT THE INVENTION

In one embodiment of the present invention, a biosensor comprises an electrode system including at least one pair of electrodes, at least one insulating base plate for supporting the electrode system, a first reaction layer provided at least on a working electrode of the electrode system and containing an organic compound which has a functional group capable of bonding or being adsorbed to an electrode and a hydrophobic hydrocarbon group, a second reaction layer provided on the first reaction layer and containing an amphiphilic lipid capable of bonding or being adsorbed to the hydrophobic portion of the first reaction layer, and a reagent system carried in a two-component membrane composed of the first and second reaction layers and containing at least membrane-binding type pyrroquinoline quinone-dependent glucose dehydrogenase and an electron mediator.

Hereinafter, the present invention will be described with reference to the accompanying drawings.

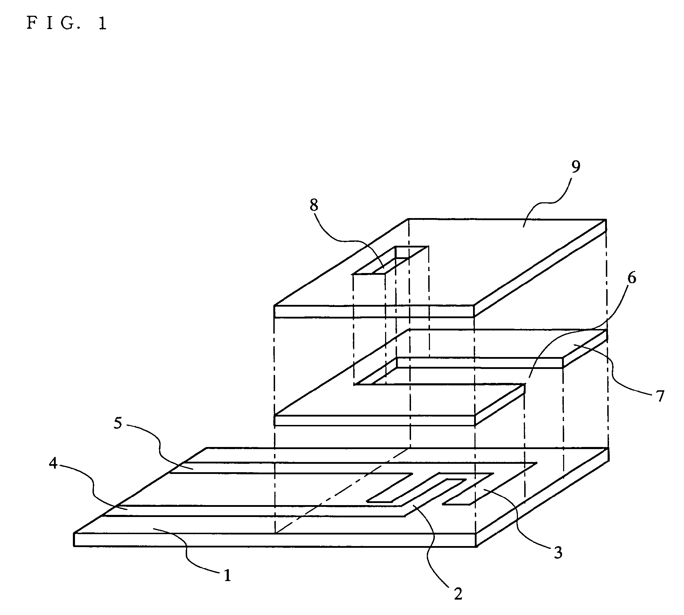

FIG. 1 is an exploded perspective view of a biosensor according to one embodiment of the present invention, where a two-component membrane and a reagent system provided therewithin are omitted. On a base plate 1, a working electrode 2 and a counter electrode 3 are provided. To form these electrodes, an electrode pattern mask made of resin or the like may be placed on the electrically insulating base plate 1, such as glass or the like. The electrode pattern mask may cover a surface of the base plate, excluding a portion of the base plate on which an electrode pattern is to be formed. Over the mask, metal such as gold or the like may be sputtered. Such a method is commonly used in the art. It should be noted that the term "working electrode" as used herein refers to an electrode (anode) which mainly causes an oxidation reaction of electron mediators, while the term "counter electrode" as used herein refers to an electrode (cathode) which mainly causes other reactions. It should also be noted that the anode and the cathode are reversed when a reduction reaction is used for detection and/or quantification of a substrate.

Alternatively, an electrode pattern may be formed on the resin base plate 1 by printing metal paste by screen printing, as described in US Patent No. 6, 340, 428. It should be noted that in order to improve adhesiveness between gold and glass, a chromium layer may be formed between gold and glass to improve tightness between the two materials. The working electrode 2 and the counter electrode 3 are electrically connected via respective leads 4 and 5 to respective measurement terminals outside the biosensor. It should be noted that the working electrode 2 and the counter electrode 3 may not be particularly distinguished from each other depending on the context in the present specification and may be simply referred to as an electrode or electrodes. As can be seen from FIG. 1, the electrode and the lead are often made of the same material and integrated together. In this case, the electrode as used herein refers to a portion of such a structure which is to be in contact with a sample solution.

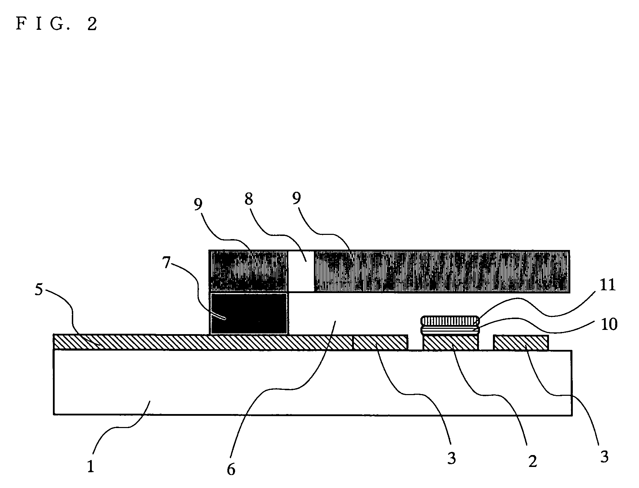

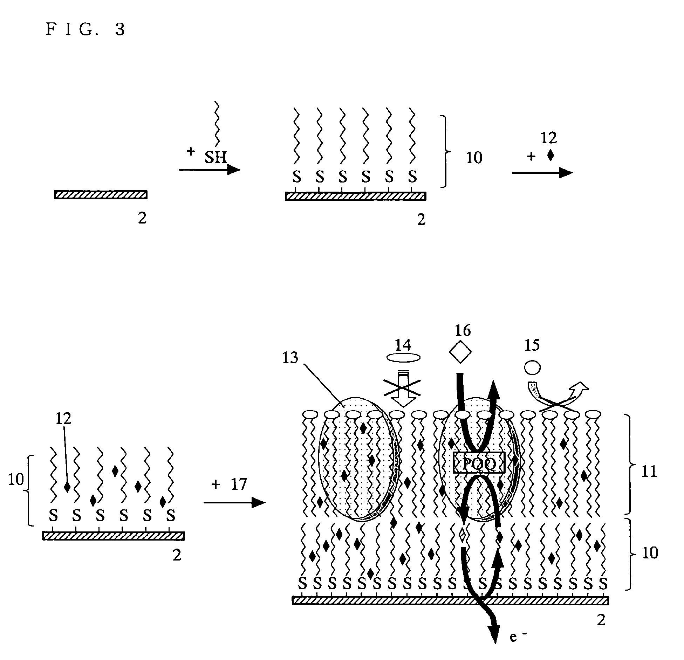

Next, the present invention will be described with reference to FIGS. 2 and 3. FIG. 2 is a longitudinal cross-sectional view of a biosensor according to one embodiment of the present invention. FIG. 3 is a schematic diagram showing a portion of a production scheme of a biosensor according to one embodiment of the present invention and an outline of the principle of the present invention.

An organic compound film 10 containing sulfur atoms is formed on a working electrode 2 formed on a base plate 1. Further, an amphiphilic lipid film 11 is formed on the organic compound film 10. An enzyme, such as PQQ-GDH, and an electron mediator, such as 1-methoxy-5-methylphenazinium, is contained as a reagent system in a two-component membrane composed of the film 10 and the film 11.

As shown in FIG. 3, the organic compound film 10 containing sulfur atoms are bound via the sulfur atoms (indicated by S in FIG. 3) to the working electrode 2. The organic compound represented by the above-described general formula has a hydrophobic portion (indicated by a single polyline in FIG. 3). The hydrophobic portion of the organic compound is preferably uniform so that a uniform two-component layer of the organic compound and the amphiphilic lipid can be formed on the base plate. In this case, a monolayer of the organic compound is easily formed on the base plate, resulting in a uniform two-component layer and a stable blocking effect. The length of a hydrocarbon chain constituting the hydrophobic portion depends on the size of n in the above-described general formula (1), (2) or (3). n is preferably an integer of 1 to 20, more preferably an integer of 5 to 15. An optimum n may be selected within the above-described ranges as appropriate in terms of removal of an interfering current due to OIC and facilitation of detection of a current based on a redox of glucose.

As an organic compound containing sulfur atoms, compounds represented by the general formula (1), (2) or (3) are preferable. Examples of such compounds include ethylthiol, propylthiol, butylthiol, pentylthiol, hexylthiol, heptylthiol, octylthiol, nonylthiol, decanethiol, undecanethiol, dodecanethiol, tridecanethiol, tetradecanethiol, pentadecanethiol, hexadecanethiol, heptadecanethiol, octadecanethiol, nonadecanethiol, icosanethiol, and disulfides of each thiol (having a structure in which the above-described thiols having the same structure are linked by S-S coupling), and thiols and disulfides whose terminal is a benzyl group, an aminobenzyl group, a carboxybenzyl group, a phenyl group or the like. These organic compounds are commercially available from suppliers well known to those skilled in the art. The above-described thiol compounds and disulfide compounds strongly tend to be adsorbed or bind strongly, irreversibly to metal surface to form substantially a monomolecule film, and are thus preferable. Moreover, a thin film made of such amonomolecule film is regularly arranged. Therefore, such regular arrangement is convenient when an amphiphilic lipid film is formed on the monomolecule film by utilizing the affinity between the film surface and the amphiphilic lipid.

Referring back to FIG. 3, after the working electrode 2 on the base plate 1 is covered with the organic compound film 10 containing sulfur atoms, the film 11 is formed. The film 11 has a single layer, in which an amphiphilic lipid having a hydrophobic portion (indicated by two polylines in FIG. 3) and a hydrophilic portion (indicated by an open ellipse in FIG. 3) is uniformly arranged. The procedure will be described below. As can be seen from FIG. 3, the hydrophobic portion of the amphiphilic lipid binds via hydrophobic interaction to the hydrophobic portion of the organic compound containing sulfur atoms, resulting in a two-component membrane (10, 11). An electron mediator 12 is distributed and included in the two-component membrane. Further, membrane-binding type PQQ-GDH binds via hydrophobic interaction to the hydrophobic portion of the two-component membrane with high affinity, so that the membrane-binding type PQQ-GDH is buried in the membrane. Although L-α-phosphatidylcholine, β-oleoyl-γ-palmitoyl is used as the amphiphilic lipid in the example, other amphiphilic lipids maybe used. The length of the hydrophobic portion is selected so that an enzyme can be buried when the two-component membrane is formed. The length depends on the size of an enzyme used and the size of its hydrophobic domain.

As described above, L-α-phosphatidylcholine, β-oleoyl-γ-palmitoyl is preferable as the amphiphilic lipid used in the present invention. L-α-phosphatidylcholine, β-oleoyl-γ-palmitoyl has two hydrophobic chains having relatively uniform lengths, so that the thickness of the amphiphilic lipid film 11 is uniform and therefore a stabler effect of blocking OIC 15 and AIC 14 is obtained. Other examples of preferable amphiphilic lipid for use in the present invention include, for example, phospholipids, such as dioleoyl, dipalmitoyl, distearoyl, dilauroyl, dimylistoyl, dilynoleoyl derivatives and the like of each of L-α-phosphatidic acid, L-α-phosphatidylcholine, L-α-phosphatidylethanolamine and L-α-phosphatidyl-DL-glycerol, or glycolipids, bile acid, and the like. These amphiphilic lipids are commercially available from suppliers well known to those skilled in the art.

With the above-described two-component membrane, the OIC 15 can be blocked completely (or substantially completely) from diffusing to the electrode surface of the biosensor. Therefore, only a series of reactions, i.e., a reaction between PQQ-GDH and glucose followed by a reaction between the electron mediator 12 and the working electrode 2, can be selectively performed. As a result, a measurement error caused by oxidation of the OIC 15 at the working electrode 2 is removed, thereby making it possible to achieve high-precision measurement. Further, the AIC 14 can be blocked completely (or substantially completely) from being adsorbed onto the surface of the working electrode 2. Furthermore, the affinity of the AIC 14 to the amphiphilic lipid 11 is significantly lower than the affinity of the AIC 14 to metal, so that a measurement error caused by adsorption of the AIC 14 on the surface of the working electrode 2 is reduced, thereby making it possible to achieve high-precision measurement.

Here, the two-component membrane used in the present invention will be further described.

Firstly, the organic compound film 10 containing sulfur atoms will be described. The sulfur atoms play a role in securing the attachment of the organic compound to the electrode. As can be seen from the above-described general formula (1), (2) or (3), the organic compound has a hydrophobic portion represented by -(CH₂)ₙ-X. As described above, the hydrophobic portion binds via hydrophobic interaction to the hydrophobic portion of the amphiphilic film 11 with high affinity, thereby helping maintain the stable two-component membrane. According to the above-described discussion, a compound for use in formation of the film 10 of the present invention is not limited to the above-described organic compound containing sulfur atoms. It will be understood that any compound having a function equivalent thereto can be used similarly. Any compound which has a functional group capable of stable attachment to the electrode and a hydrophobic portion capable of binding to amphiphilic lipid with high affinity can be used for the formation of the two-component membrane of the present invention. Further, as described above, such a compound having a function equivalent thereto preferably has a structure which allows the formation of a monolayer on the electrode surface. Thereby, it is possible to obtain a uniform layer thickness and a stabler OIC blocking effect. However, the film 10 is not necessarily a monolayer. For example, in the film 10, the functional groups of a portion of the organic compoundmolecules may not be in contact with the electrode, and the hydrophobic portions thereof may be inserted into gaps in the substantial monolayer. Alternatively, the whole layer of the film 10 may have a wavelike structure or a part or the whole layer of the film 10 may have a multilayer, due to the surface roughness of the base plate and the electrode. Even in such cases, the effect of the present invention can be considered to be obtained. As used herein, the term "first reaction layer" is used to refer to a concept comprehensively including the organic compound film 10 containing sulfur atoms, which is formed on the electrode, as well as a film having a function equivalent thereto.

Next, the amphiphilic lipid film 11 formed on the film 10 will be described. The hydrophobic portion of an amphiphilic lipid plays a role in binding via hydrophobic interaction to the hydrophobic portion of the compound constituting the first reaction layer with high affinity. In this case, the film 11 is not necessarily a monolayer as shown in FIG. 3. For example, in the film 11, the tips of the hydrophobic groups of a portion of the amphiphilic lipid molecules may not be in contact with the film 10, and the hydrophobic portion of the molecule may be inserted into gaps in the substantial monolayer. Alternatively, the whole layer of the film 11 may have a wavelike structure or a part or the whole layer of the film 11 has a multilayer, due to the surface roughness of the base plate and the electrode or an influence of the structure of the film 10. Even in such cases, the effect of the present invention can be considered to be obtained. As used herein, the term "second reaction layer" is used to refer to a concept comprehensively including the amphiphilic lipid film 10 provided on the first reaction layer as well as a film having a function equivalent thereto.

The term "two-component membrane" briefly refers to a concept indicating what is composed of a first reaction layer and a second reaction layer as described above on an electrode. Representatively, as schematically shown in FIGS. 2 and 3, the two-component membrane provides an environment similar to that of a lipid bilayer, which is seen in cell membrane. Therefore, a membrane-binding type enzyme can be buried in the two-component membrane as can be seen in naturally-occurring cell membranes or the like. It should be noted that the film 10 and the film 11 do not have to be a monolayer as described above, and therefore, the two-component membrane does not have to be a two-molecule layer composed of a combination of two monolayers as shown in FIG. 3. Further, the organic compound molecules having a functional group and the amphiphilic lipid molecules may be complexly mixed and distributed in a vicinity of a boundary portion between the first reaction layer and the second reaction layer. The two-component membrane of the present invention achieves a function of effectively blocking the influence of OIC on a redox reaction caused by glucose on the electrode surface and the influence of AIC adsorbed on the electrode surface, and a function of selectively performing only a redox reaction, in which glucose is involved, between the two-component membrane and the electrode. In the present invention, the two-component membrane further contains the electron mediator 12 for achieving electron transport between the enzyme and the working electrode 2.

As the electron mediator 12 used in the present invention, a compound which achieves electron transport from PQQ-GDH to the working electrode 2 is employed. As the electron mediator 12 used in the present invention, a compound such as 1-methoxy-5-methylphenazinium is preferable. This is because the planarity of the molecule is high so that the molecule is inserted inside of the membrane and is located near the working electrode, and therefore, efficient electron transfer is achieved. Further, phenazine derivatives, phenothiazine derivatives (e.g., azure, thionine, etc.), quinone derivatives and the like similarly have a high level of molecular planarity and are thus preferable. These compounds are commercially available from suppliers well known to those skilled in the art.

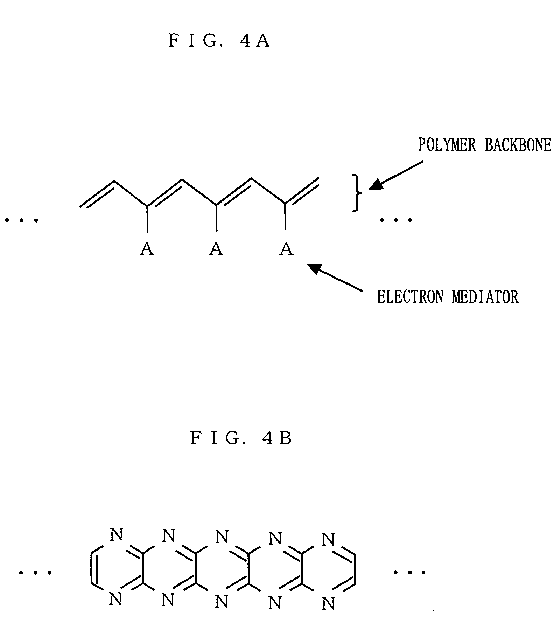

Further, the electron mediator 12 may be linked with a polymer backbone (see FIG. 4A), or alternatively, a part or the whole of the electron mediator 12 may constitute a polymer chain (see FIG. 4B). Such an electron mediator is commercially available from suppliers well known to those skilled in the art.

One, or two, or more electron mediators 12 are used. These are only examples. The electron mediator 12 for use in the implementation of the present invention is not limited to the above-described examples.

Next, it will be described how the two-component membrane is formed on the electrode.

As schematically shown in FIG. 2, substantially the entire surface of the working electrode 2 is preferably covered with the organic compound film 10 containing sulfur atoms. In order to cover the working electrode 2 with the organic compound film 10 containing sulfur atoms, the surface of the working electrode 2 is immersed in a solution of the above-described organic material, or alternatively, such a solution is dropped onto the surface of the working electrode 2. Alternatively, similar covering can be achieved by exposing the surface to the vapor of the organic material. However, when the working electrode 2 and the counter electrode 3 are disposed on the same base plate as shown in FIGS. 1 and 2, only the working electrode surface may be covered, or alternatively, a portion of the counter electrode surface and the entire or substantially the entire of the working electrode surface may be covered. To achieve this, a region to be covered with the organic compound solution is accurately defined, preferably as follows.

Specifically, arod-likeresinmemberhavinga surface, which has substantially the same shape as that of the working electrode 2 and optionally the counter electrode 3 and can form a desired contact area to the electrode(s), is used. A small amount of solution containing the above-described organic compound is applied to a surface of the member. The surface of the member is made contact with a desired region including a surface of the working electrode 2 and optionally the counter electrode 3. Thereby, the organic compound solution applied on the surface of the member can be transferred onto the surface of the working electrode 2. In this manner, a region on the electrode to be covered with the organic compound film is precisely defined so that the measurement precision of a biosensor can be maintained. Such a so-called transfer method is commonly used in the art and well known to those skilled in the art.

Further, the organic compound film 10 containing sulfur atoms, which covers the working electrode surface, is covered with amphiphilic lipid as follows. The working electrode, whose surface is covered with the organic compound film containing sulfur atoms, is immersed in a dispersed solution of amphiphilic lipid vehicles (liposomes).

In order to bury PQQ-GDH and the electron mediator in the amphiphilic lipid film, it is effective to cause the PQQ-GDH and electron mediator to exist in the solutions in the respective film formation steps.

As a sample solution containing a substrate to be measured in practicing the present invention, a sample solution containing glucose is preferable. Examples of such a sample solution include biological sample solutions, such as blood, plasma, serum, cell interstitial fluid, saliva, urine and the like, or food and beverages, and the like. In addition,electrolyticsolutionsused in a typical level of laboratories, liquids used for environmental assessment and the like can be employed. Particularly when glucose is measured, solutions containing glucose as well as OIC and AIC (e.g., whole blood, plasma, urine, etc.) are often used as sample solutions.

Any indicator can be herein used for measurement of a substrate contained in a sample solution as long as it is output due to a change in electrochemical reaction. Examples of such an indicator include a current or an amount of electrical charges passed.

In the biosensor of the present invention, the working electrode 2 preferably contains gold, platinum or palladium. In this case, a potential applied to the working electrode 2 is stabilized, thereby achieving higher-precision measurement.

In order to cause the organic compound containing sulfur atoms to be securely adsorbed on the working electrode 2, the working electrode 2 preferably contains noble metal, such as gold, palladium, platinum or the like, or other transition metals, such as silver, copper, cadmium or the like. Therefore, in order to cover substantially only the working electrode 2 with the organic compound film 10, it is preferable that the counter electrode 3 contains none of these metals.

Further, it is preferable that at least a portion of the surface of the counter electrode 3 is not covered with the organic compound film 10 containing sulfur atoms or the amphiphilic lipid film 11. In this case, a material to be reduced, which is contained in a sample solution, reaches a portion of the surface of the counter electrode 3, which is not covered with the film, more easily than a portion thereof covered with the film. In the portion not covered with the film, therefore, a reduction reaction (cathode reaction) proceeds more easily at the counter electrode 3, thereby making it possible to perform more stable measurement. The area of the portion of the counter electrode 3, which is not covered with the organic compound film 10 containing sulfur atoms or the amphiphilic lipid film 11, is preferably larger than the area of the working electrode 2. Further, it is preferable that substantially no organic compound film 10 containing sulfur atoms or amphiphilic lipid film 11 exist on the counter electrode 3. Furthermore, it is preferable that substantially only the working electrode 2 is covered with the organic compound film 10 containing sulfur atoms and the amphiphilic lipid film 11. In this case, a reduction reaction proceeds more smoothly at the counter electrode 3, thereby making it possible to perform more stable measurement.

Referring to FIG. 1, a spacer 7 having a slit 6 and a cover 9 having an air hole 8 are adhered to on the base plate 1 as prepared above in a positional relationship as indicated with a dash dot line in FIG. 1. Thus, a biosensor of the present invention is fabricated. A sample solution supply path is formed in a portion of the slit 6 of the spacer 7. An open end of the slit 6 at an end of the biosensor functions as a sample solution supply inlet toward the sample solution supply path.

When a sample solution is caused to be in contact with the open end of the slit 6 which functions as the sample solution supply path in the biosensor having the structure of FIG. 1, the sample solution is introduced into the sample solution supply path due to capillary phenomenon, and an action of the reagent system causes an enzyme reaction to proceed. Thus, when the cover member composed of the spacer 7 and the cover 9 is combined with the base plate 1 equipped with the electrode system to form the sample solution supply path, a constant amount of sample solution containing a substrate to be measured can be supplied to the biosensor, thereby making it possible to improve measurement precision. Further, a pH buffering agent can be provided at the cover member side in the biosensor equipped with the sample solution supply path.

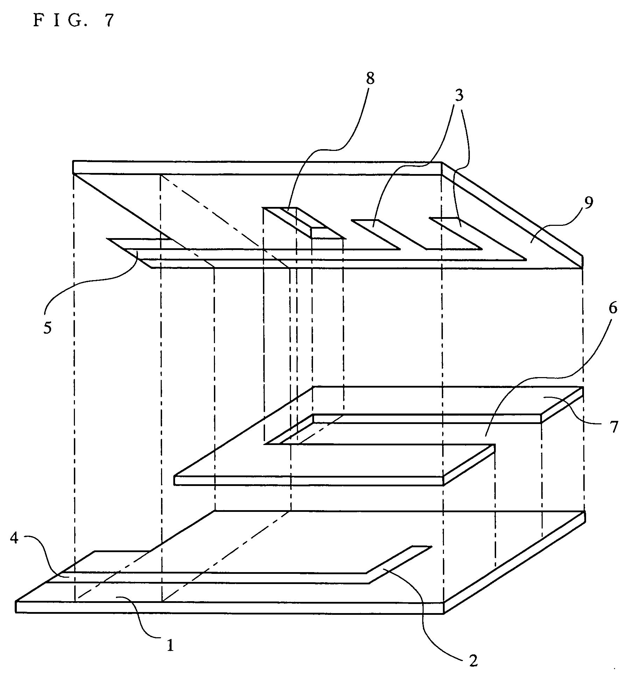

Further, as shown in FIG. 7, a second insulating base plate, on which either the counter electrode 3 or the working electrode 2 is formed along with a corresponding lead 5 or 4, may be used instead of the cover 9. In this case, a sample solution supply path is formed by the base plate 1, the spacer 7 and the second base plate, so that a constant amount of sample solution can be supplied to the biosensor, thereby making it possible to improve measurement precision. An exemplary biosensor having such a form is described in, for example, US Patent No. 6,458,258.

Alternatively, a biosensor can be constructed using only the base plate 1 without forming the above-described sample solution supply path. In this case, a reagent system is provided on or near the electrode system. An exemplary biosensor having such a form is described in, for example, the above-described Japanese Laid-Open Patent Publication No. 3-202764.

Hereinafter, the present invention will be described by way of examples. These examples are only for illustrative purposes but do not limit the present invention.

### (Example 1)

### Fabrication of a biosensor of the present invention

### a. Preparation of proteosome suspension

The following preparation method is only an example. The present invention is not limited to this.

Firstly, a liposome of L-α-phosphatidylcholine, β-oleoyl-γ-palmitoyl (hereinafter referred to as PCOP) (available from Wako Pure Chemicals) was prepared as follows. PCOP was dissolved in trichloromethane within a round-bottom flask to a concentration of 10 mM. A rotary evaporator was used to evaporate the solvent completely under reduced pressure. Next, the PCOP was dissolved again in the flask using 2-propanol to a PCOP concentration of 40 mM. 0.5 mL of the resultant solution was added to 10 mL of 20 mM Tris-HCl buffer solution (pH=7.3) containing 0.15 M NaCl. The solution was agitated strongly for 10 min to obtain a PCOP liposome suspension.

Next, in order to incorporate an enzyme and an electron mediator into the liposome, 1 mg of PQQ-GDH (prepared from *Acinetobacter calcoaceticus* as described in the above-mentioned Matsushita et al. (1989)) and 30 mg of 1-methoxy-5-methylphenazinium (hereinafter abbreviated as MMP) (available from Dojindo Laboratories) as the electron mediator were added to 10 mL of the PCOP liposome suspension, followed by strong agitation for 10 min. The thus-obtained PCOP liposome suspension containing PQQ-GDH and MMP is hereinafter referred to as an MMP-proteosome suspension.

### b. Preparation of a base plate

In order to fabricate a working electrode and a counter electrode on a base plate, firstly, an electrode pattern mask made of resin was provided on the electrically insulating base plate 1 made of glass. Over the resultant structure, chromium was sputtered to form a chromium layer. Further, gold was sputtered over the chromium layer. Thus, the working electrode 2 and the lead 4, and the counter electrode 3 and the lead 5 were formed.

### c. Electrode treatment

The thus-formed working electrode 2 was covered with n-octylthiol (hereinafter referred to as OT) in accordance with the above-described transfer method as follows. Firstly, a small amount of OT ethanol solution (concentration: 5 mM) was applied to a planar surface of a rod-like instrument made of resin, the planar surface having substantially the same shape as that of the working electrode 2 on the base plate 1. Next, the position of the OT-applied surface was carefully adjusted to overlap substantially the entire surface of the working electrode 2. Thereafter, the OT-applied surface was laid on the surface of the working electrode 2. Next, the rod-like instrument was drawn away from the working electrode 2 in a manner which allowed the OT solution to remain on the working electrode 2. The working electrode 2 was allowed to stand until OT was adsorbed onto the working electrode surface.

As a result, the organic compound film 10 (i.e., OT film), which contains sulfur atoms in molecules constituting the film, was formed. After one hour, ethanol and ultrapure water were used sequentially to rinse the working electrode 2. The resultant base plate 1 was immersed in 10 mM MMP aqueous solution for one hour, causing MMP to enter into the OT film. After sufficiently rinsed using ultrapure water, the base plate 1 was immersed in the above-described MMP-proteosome suspension for eight hours, so that MMP-proteosome 17 was fused with the OT film 10 on the working electrode 2 (see FIG. 3).

The base plate 1 was rinsed using ultrapure water. After drying, the spacer 7 and the cover 9 was combined with the base plate 1 to fabricate a biosensor as shown in FIG. 2.

Also, a comparative example was fabricated as follows. One microliter of solution obtained by dissolving 1 mg of PQQ-GDH and 30 mg of MMP in 10 mL of 20 mM Tris-HCl buffer solution (pH=7. 3), was dropped on the surface of the working electrode 2 on the base plate 1, followed by drying. The spacer 7 and the cover 9 were combined with the base plate 1 to fabricate a biosensor.

### (Example 2)

### Influence of AIC on the biosensor of the present invention

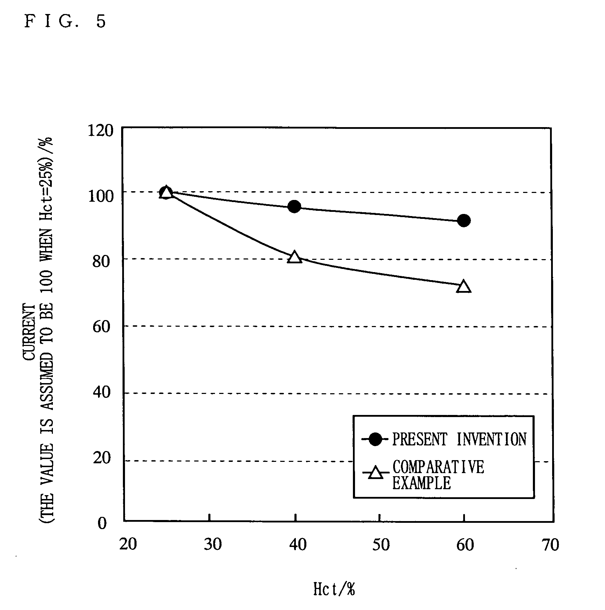

Blood containing a predetermined amount of D-glucose (400 mg/dL) was supplied as a sample solution to an opening portion of a sample solution supply path (i.e. , the open end of the slit 8 of the spacer 7) of each of the biosensor prepared above according to one embodiment of the present invention and the biosensor of the comparative example. It should be noted that sample solutions having different red blood cell volume ratios (hematocrit (hereinafter abbreviated as Hct)), i.e., 25, 40 and 60%, were used. After a predetermined time (reaction time: 25 sec) had passed, a voltage of 500 mV was applied to the working electrode 2 with respect to the counter electrode 3. Five seconds after that, a flowing current value was measured. As shown in FIG. 5, in the case of the biosensor of the comparative example, it was observed that the current tended to be decreased with an increase in Hct.

This result suggests that the amount of red blood cells adsorbed on the working electrode surface tends to be increased with an increase in Hct, and corresponding to the tendency, an electrode reaction is inhibited. As a result, it is considered that the current value varied depending on the presence of Hct, resulting in measurement error, although glucose concentration was the same.

By contrast, in the biosensor of this example, substantially the same current values were obtained irrespective of the presence of Hct. Therefore, it is considered that the adsorption of red blood cells on the surface of the working electrode 2 was suppressed by the OT and PCOP films provided on the surface of the working electrode 2. A physical property of the electrode surface covered with the films of OT and PCOP (organic compounds) is significantly altered from an uncovered gold surface. Alternatively, an interface of the electrode is charged positively or negatively due to a terminal group of the covering film.

It can be considered that the effect of both or either of these alterations is responsible for suppression of adsorption of blood cells. In addition, it was found that MMP incorporated into the OT and PCOP films has a function of achieving electron transport between PQQ-GDH and the working electrode. Thus, by covering the working electrode with the OT and PCOP films, it was possible to remove measurement errors due to adsorption of AIC.

### (Example 3)

### Influence of OIC on the biosensor of the present invention

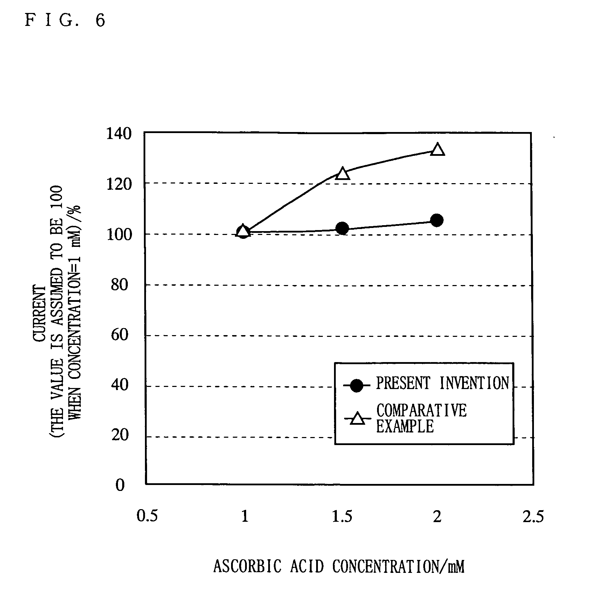

Ascorbic acid, which is an OIC, was added to blood having a Hct of 40% so that the total of the amount of the additional ascorbic acid and the amount of ascorbic acid contained in the original blood was adjusted to a concentration of 1, 1.5 and 2 mM. These blood samples were used to measure current values as described above. As shown in FIG. 6, the biosensor of the comparative example tended to have an increase in current with an increase in ascorbic acid concentration. This suggests that an oxidation reaction of ascorbic acid proceeds at the working electrode. As a result, it is considered that the current value varied depending on ascorbic acid concentration, resulting in measurement error, although glucose concentration was the same.

In contrast, the biosensor of this example obtained substantially the same current value irrespective of ascorbic acid concentration. The reason is considered to be that the gold electrode surface of the working electrode 2 was covered with the OT and PCOP films made of organic compounds having a long molecule chain length, so that the film prevented ascorbic acid from approaching the gold electrode surface. Thus, by covering the working electrode with the OT and PCOP films, it was possible to remove errors due to electrochemical oxidation of OIC as well as measurement errors due to AIC adsorption.

### (Example 4)

### Use of a reference electrode

In this example, immediately after the sample solution was supplied to a biosensor according to one embodiment of the present invention, which was fabricated with a procedure similar to that as described in Example 1, a silver/silver chloride electrode was made contact with a sample solution near the sample solution supply inlet via a salt bridge made of potassium chloride and agar. The silver/silver chloride electrode has a stable potential and can be used as a reference electrode.

Blood samples having various Hct values, which contained a predetermined amount of D-glucose (400 mg/dL), were supplied as sample solutions to the opening portion of the sample solution supply path of the biosensor (i.e., the open end of the slit 8 of the spacer 7). After 25 seconds had passed, a voltage of 500 mV was applied to the working electrode 2 with respect to the silver/silver chloride electrode. After five seconds, a flowing current value was measured.

As a result, substantially the same current values were obtained irrespective of Hct. Under the same conditions, variations in the current value were smaller compared to the result of Example 2. Therefore, it was found that the stability of measured values was improved by introducing a reference electrode to the biosensor system.

Blood samples having a Hct of 40% and an ascorbic acid concentration of 1, 1.5 and 2 mM were used to measure current values in a manner similar to that described above. As a result, substantially the same current values were obtained irrespective of ascorbic acid concentration. Under the same conditions, variations in the current value were smaller compared to the result of Example 3. Therefore, it was found that the stability of measured values against variations in ascorbic acid concentration was improved by introducing a reference electrode to the biosensor system.

### (Example 5)

### Use of n-octyl disulfide

In this example, n-octyl disulfide was used instead of OT to fabricate a biosensor using the method described in Example 1. A response to glucose in blood was measured in a manner similar to that described in Examples 2 and 3.

As a result, also in this example, substantially the same current values were obtained irrespective of Hct and ascorbic acid concentration as in Examples 2 and 3. OT is a compound which is obtained by splitting the S-S bond of n-octyl disulfide. It is known that thiol and disulfide having such a correspondence form similar films.

### (Example 6)

### A working electrode and a counter electrode formed on different base plates

In this example, the counter electrode 3 was formed on the cover 9. The surface of the working electrode 2 was modified as described in Example 1. A response to glucose in blood was measured in a manner similar to that described in Examples 2 and 3.

As a result, also in this example, substantially the same current values were obtained irrespective of Hct and ascorbic acid concentration as in Examples 2 and 3. Therefore, it was found that when electrodes are formed on a plurality of base plates, a similar effect is obtained. In this example, the working electrode 2 and the counter electrode 3 were located on different base plates. Therefore, in the fabrication process of the sensor, substantially only the working electrode 2 can be covered with a film without covering the counter electrode 3 with the film by immersing the base plate 1 having the working electrode 2 in OT solution or exposing the surface of the working electrode 2 on the base plate 1 to OT vapor. Thus, according to this example, a sensor in which substantially only the working electrode is covered can be easily fabricated as compared to a sensor in which the working electrode and the counter electrode are provided on the same surface.

### (Example 7)

### Use of platinum or palladium

In this example, platinum or palladium was used to fabricate the working electrode 2 and the counter electrode 3. Each electrode was formed by providing an electrode pattern mask made of resin on the electrically insulating base plate 1 made of glass, forming a chromium layer, and sputtering platinum or palladium. Further, the surface of the working electrode 2 was covered in a manner similar to that described in Example 1. A response to glucose in blood was measured in a manner similar to that described in Examples 2 and 3.

As a result, when platinum was used, substantially the same current values were obtained irrespective of changes in ascorbic acid concentration, though the current values were somehow dependent on Hct. By contrast, in a biosensor fabricated in a manner similar to that of the comparative example described in Example 1 where platinum was used instead of gold, current was more significantly dependent on Hct and ascorbic acid concentration.

According to the result, it was found that when platinum is used as a material for the working electrode, the effect of suppressing the influence of OIC and AIC can also be obtained by covering the working electrode with the membrane of the present invention. Further, it was found that when palladium is used as a material for the working electrode, the independence of current from Hct and ascorbic acid concentration was observed to as high an extent as when gold is used. It was found that palladium is also a considerably preferable electrode material in the present invention.

### (Example 8)

### Effect of a pH buffering agent

Biosensor characteristics were evaluated when a pH buffering agent was further included in a biosensor system. The biosensor prepared in this example was similar to that used in Example 1, except that a mixture of dipotassium hydrogen phosphate and potassium dihydrogen phosphate was carried as a pH buffering agent in the amphiphilic lipid film 11 containing PQQ-GDH and an electron mediator as a reagent system.

Blood samples having various Hct values, which contained a predetermined amount of D-glucose (400 mg/dL), were supplied as sample solutions to a space portion of the biosensor. After a predetermined time had passed, a voltage of 500 mV was applied to the working electrode 2 with respect to the counter electrode 3, the flowing current value was measured. As a result, the resultant current value was not dependent on Hct.

Comparing the above result with the results of Examples 2 and 3, the dependency of the current value on ascorbic acid concentration was substantially the same and the dependency on Hct was further reduced. Thus, a constant current value more independent from Hct was obtained where glucose concentration was the same.

The reason such a result was obtained is considered as follows. By providing a pH buffering agent in the biosensor system, the pH of a sample solution in the biosensor is stabilized. Therefore, the charge state of a terminal group of the membrane provided on the electrode is stabilized, so that the effect of preventing adsorption of AIC in blood is made constant for each sample solution.

Further, it is considered that the stable pH also stabilized the activity of the enzyme, so that the amount of reduced electron mediators after a predetermined time was made constant for each sample solution. It is considered that both or one of the two stabilization effects caused by stabilization of pH reduced the dependency of the current value on Hct. When the pH of a measured solution was 4 to 9, the stabilization effect was particularly observed in this example. Therefore, a preferable pH region is pH 4 to 9. A more preferable pH region is a pH range of 5 to 8 in terms of the stablest enzyme activity.

Although a current value was measured in the above-described Examples 2 to 8, a tendency similar to that of the current value was observed when a charge amount was measured instead of the current value.

Also in the above-described examples, a voltage of 500 mV was applied to the electrode system. However, the applied voltage is not limited to that value. Any voltage is applied as long as it causes the electron mediator to be oxidized at the working electrode.

In the above-described examples, the reaction time was 25 sec or 55 sec and the voltage application time were 5 sec after the reaction time. The present invention is not limited to this. Any times can be used as long as an observable current can be obtained.

Further, the reagent system, or one or more reagents contained in the reagent system are optionally fixed on the working electrode, so that an enzyme and an electron mediator are preferably caused not to be dissolved or eluted. In this case, a method utilizing interaction with the membrane via van der Waals force, a covalent bond method, a cross-linking fixation method, or a fixation method using coordinate bond or specific binding interaction, can be used. Particularly in practicing the present invention, it is also preferable to fix the reagent via covalent bond to the organic compound film containing sulfur atoms on the electrode.

It has been described that membrane-binding type PQQ-GDH is a preferable enzyme in practicing the present invention. However, it is not intended that the enzyme used in the present invention is limited only to membrane-binding type PQQ-GDH. Any enzyme other than membrane-binding type PQQ-GDH, which has a hydrophobic domain in at least a portion of the molecule surface thereof and can bind to the hydrophobic portion of a two-component membrane as described above with high affinity (or an enzyme which can be buried in a two-component membrane) and which catalyzes a selective oxidation reaction of glucose, may be used similarly. A preferable combination of components constituting the two-component membrane may vary depending on the enzyme.

By including an enzyme other than PQQ-GDH in the reagent system, a substrate other than glucose can be measured. For example, by adding invertase having a function of degrading sucrose to glucose and fructose to the reagent system, quantification of sucrose can be performed.

In the above-described examples, a sputtering method using a mask was employed to fabricate electrodes and their patterns. The present invention is not limited to this. For example, a metal film, which is produced using any of sputtering, ion plating, vapor deposition, and chemical vapor deposition, may be used in combination with photolithography and etching to produce a pattern. Pattern formation can also be performed by trimming metal using laser. Screen printing may be performed using metal paste on a base plate to form an electrode pattern. Alternatively, a patterned metal foil may be adhered directly to an insulating base plate.

The shapes, arrangements, quantities and the like of these electrode systems are not limited to those of the above-described examples. For example, the working electrode and the counter electrode may be formed on different insulating base plates (see FIG. 7). A plurality of each of the working electrode and the counter electrode may be formed. Also, the shapes, arrangements, quantities and the like of the leads and the terminals are not limited to those of the above-described examples.

In order to improve measurement precision, a spacer is preferably included as a component in the above-described biosensor. This is because the amount of a solution containing a substrate to be measured can be easily set to a predetermined amount. However, when the biosensor of the present invention is used in combination with an instrument capable of collecting a predetermined volume of sample solution, a cover member composed of a spacer and a cover is not necessarily required. Materials for these spacer, cover or base plate are not limited only to glass which is used as a material for the base plate in the above-described examples. Other materials may be used in implementation of the biosensor of the present invention, including, for example, electrically insulating materials, such as inorganic materials (e.g., silicon and an oxide thereof, etc.), resins (e.g., polyethylene terephthalate (PET), polypropylene (PP), etc.), and the like.

### INDUSTRIAL APPLICABILITY

As described above, the biosensor of the present invention is suitable as a simple structure biosensor capable of measuring a substrate contained in a sample solution quickly and with high precision, without an influence of AIC and OIC contained in the sample solution. Particularly, the biosensor of the present invention is suitable as a disposable biosensor for detecting the glucose concentration of a sample solution.

## Claims

1. A biosensor, comprising:
an electrode system including at least one pair of electrodes;
at least one insulating base plate for supporting the electrode system;
a first reaction layer provided at least on a working electrode of the electrode system, including an organic compound having a functional group capable of bonding or being adsorbed to an electrode and a hydrophobic hydrocarbon group;
a second reaction layer provided on the first reaction layer, including an amphiphilic lipid capable of bonding or being adsorbed to a hydrophobic portion of the first reaction layer; and
a reagent system carried in a two-component membrane composed of the first and second reaction layers, including at least membrane-binding type pyrroquinoline quinone-dependent glucose dehydrogenase and an electron mediator.

2. The biosensor according to claim 1, wherein the first reaction layer and the second reaction layer each form a monolayer.

3. The biosensor according to claim 1, wherein the organic compound is a compound represented by the following general formula (1), (2) or (3);
HS-(CH₂)ₙ-X (1)
X-(CH₂)ₙ-S-S-(CH₂)ₙ-X (2)
S-(CH₂)ₙ-X (3)
where n is an integer of 1 to 20, and X is a methyl group, a benzyl group, an aminobenzyl group, a carboxybenzyl group or a phenyl group.

4. The biosensor according to claim 1, wherein the amphiphilic lipid is L-α-phosphatidylcholine, β-oleoyl-γ-palmitoyl.

5. The biosensor according to claim 1, wherein the electron mediator is 1-methoxy-5-methylphenazinium.

6. The biosensor according to claim 1, wherein the working electrode contains gold, platinum or palladium.

7. The biosensor according to claim 1, wherein a counter electrode of the electrode system contains none of gold, platinum and palladium.

8. The biosensor according to claim 1, wherein the entirety of a surface of the working electrode of the electrode system and a portion of a surface of a counter electrode are covered with the two-component membrane.

9. The biosensor according to claim 8, wherein the area of a portion of the counter electrode, which is not covered with the two-component membrane, is larger than the area of the working electrode.

10. The biosensor according to claim 1, wherein only the surface of the working electrode of the electrode system is covered with the two-component membrane.

11. The biosensor according to claim 1, wherein the pair of electrodes are supported on respective surfaces of two insulating base plates, the surfaces of the two insulating plates facing each other in a manner which forms a supply path for the sample solution therebetween.

12. The biosensor according to claim 1, wherein the reagent system further includes a pH buffering agent.

13. A method for fabricating a biosensor, comprising the steps of:
forming an electrode system including at least one pair of electrodes on an insulating base plate;
providing a first reaction layer at least on a working electrode of the electrode system, wherein a solution containing an organic compound having a functional group capable of bonding or being adsorbed to an electrode and a hydrophobic hydrocarbon group is made contact with the working electrode; and
providing a second reaction layer on the first reaction layer, wherein a solution containing an amphiphilic lipid is made contact with the first reaction layer,
wherein at least one of the solution containing the organic compound and the solution containing the amphiphilic lipid contains an electron mediator, and the solution containing the amphiphilic lipid further contains membrane-binding type pyrroquinoline quinone-dependent glucose dehydrogenase.

14. A method according to claim 13, wherein the first reaction layer and the second reaction layer each form a monolayer.

15. A method according to claim 13, wherein the organic compound is a compound represented by the following general formula (1), (2) or (3);
HS-(CH₂)ₙ-X (1)
X-(CH₂)ₙ-S-S-(CH₂)ₙ-X (2)
S-(CH₂)ₙ-X (3)
where n is an integer of 1 to 20, and X is a methyl group, a benzyl group, an aminobenzyl group, a carboxybenzyl group or a phenyl group.

16. A method according to claim 13, wherein the amphiphilic lipid is L-α-phosphatidylcholine, β-oleoyl-γ-palmitoyl.

17. A method according to claim 13, wherein the electron mediator is 1-methoxy-5-methylphenazinium.

18. A method according to claim 13, wherein the working electrode contains gold, platinum or palladium.

19. A method according to claim 13, wherein a counter electrode of the electrode system contains none of gold, platinum and palladium.

20. A method according to claim 13, wherein the entirety of a surface of the working electrode of the electrode system and a portion of a surface of a counter electrode are covered with the first and second reaction layers.

21. A method according to claim 20, wherein the area of a portion of the counter electrode, which is not covered with the first and second reaction layers, is larger than the area of the working electrode.

22. A method according to claim 13, wherein only the surface of the working electrode of the electrode system is covered with the first and second reaction layers.

23. A method according to claim 13, wherein the pair of electrodes are supported on respective surfaces of two insulating base plates, the surfaces of the two insulating plates facing each other in a manner which forms a supply path for the sample solution therebetween.

24. A method according to claim 13, wherein at least one of the solution containing the organic compound and the solution containing the amphiphilic lipid further contains a pH buffering agent.
